# EUROPEAN PATENT APPLICATION

(11) **EP 1 393 765 A1**
(43) Date of publication of application: **03.03.2004**
(21) Application number: 03077649.6
(22) Date of filing: 25.08.2003
(51) Int. Cl.: A61M 5/32, A61B 5/15

(54) **Needle holder**

(30) Priority: 29.08.2002 US 230507
(71) Applicant: Becton, Dickinson and Company, Franklin Lakes, New Jersey 07417-1880 (US)
(72) Inventor: Ward, William E. Jr., Sumter, South Carolina 29150 (US)
(74) Representative: Wittop Koning, Tom Hugo

(57) **Abstract**

The present invention provides a needle holder including an elongate tubular body having an open end for receiving a specimen collection tube, an opposed closed end for supporting a needle, and a cylindrical wall therebetween. A rotatable finger grip flange is supported about the cylindrical wall at the open end of the body. The tubular body further includes a radially outwardly extending flanged portion at the open end and at least one outwardly extending shoulder which is spaced from the flanged portion for longitudinally captivating the finger grip flange therebetween. The rotatable finger grip flange allows the user to properly orient the needle bevel of a needle supported on the needle holder to obtain maximum capability of piercing a patient's skin.

## Description

### BACKGROUND OF THE INVENTION

### 1. Field of the Invention

The present invention relates generally to a needle holder for supporting a specimen collection tube and, more particularly, the present invention relates to a needle holder with a rotatable finger grip.

### 2. Description of Relevant Art

Blood samples and other medical specimens are routinely taken and collected in a specimen collection container. In blood collection applications, the collection container is typically a hollow blood collection tube with one end closed by a semi-spherical portion and the other end open. The open end is sealable by an impervious elastomeric cover. The tube thus defines an interior chamber for collecting and holding the blood sample.

To collect a blood sample, the tube is used in concert with a tube holding device, commonly known as a "needle holder". This device typically comprises a hollow needle extending through the closed end of a tubular housing. The tubular housing has an opposed open end for accepting the blood collection tube. While grasping a finger flange located at the open end of the tubular housing, the phlebotomist inserts the hollow needle through the dermal layer of a subject into the lumen of the blood vessel and taps into the circulation system of a subject to direct blood therethrough towards the interior compartment of a holding device. The collection container is inserted through the open end of the holder so that the hollow needle punctures through the cover of the collection container.
The interior of the collection container is now in direct communication with the circulation system of a patient and, having typically been formed in a vacuum, draws blood through the hollow needle and into the collection container. Once the phlebotomist has drawn enough blood into the collection container, the container may be removed from the holder. The container cover, being of an elastomeric material, reseals the hole made by the needle. The phlebotomist may then, if desired, insert a new collection container into the holder to draw more blood.

The hollow needle of the needle holder includes a beveled end. For proper handling of the needle holder when piercing the patient's skin, it is known that the beveled plane of the needle should be oriented with respect to the finger grip flange at the open end of the needle holder, with the needle bevel facing away from the skin.

Using a needle holder comprised of a fixed finger flange, a phlebotomist would need to follow the following steps: (a) remove the protective sheath on the needle; (b) note the orientation of the bevel of the needle tip relative to the orientation of the finger grip flange; (c) replace the protective sheath on the needle; (d) rotate the sheath and needle combination empirically so as to attempt to properly position the needle bevel; (e) again remove the sheath on the needle to verify the orientation of the needle bevel with respect to the finger grip flange; and (f) repeat the steps above until a satisfactory orientation of the needle bevel with respect to the orientation of the finger grip flange has been achieved. This satisfactory orientation, as well known in the field, provides a large improvement in the skin-end blood vessel membrane-piercing capability of the needle.

As can be appreciated, the steps above result in loss of time, stress sustained by the phlebotomist and an increase in the likelihood that the phlebotomist will contaminate the needle with a finger or be punctured by the needle during the replacement of the sheath thereon.

It is known to use a rotatable finger grip which allows the user to align the bevel of a needle relative to the skin. For example, a syringe is known that allows for rotational movement of a winged finger grip around the body of the syringe in order to orient the needle bevel with respect to the longitudinal axis of a winged finger grip. The rotatable finger grip is retained in association with the body of the syringe by a snap-fit into the syringe. This finger grip includes a circumferential groove on an internal surface which mates with a fixed notched flange at the open mouth of the syringe to help provide the snap-fit. In order to effect rotation of the finger grip about the body of the syringe, the user must radially compress the syringe at the fixed flange so as to reduce temporarily the diameter of the flange. The notches of the fixed flange provide the radial flexibility which permits this temporary reduction in the diameter. The needle sheath is removed as the user effects rotation. The disadvantage of this device is that it requires the phlebotomist to apply forceable action to the syringe with both hands. In particular, the phlebotomist applies forcible action to the syringe body with one hand, while simultaneously using a thumb and forefinger from the other hand to forcibly rotate the finger grip. This method is cumbersome, resulting in loss of time and an increased likelihood that the phlebotomist will be injured by the unsheathed needle while rotating the finger grip.

Therefore, there is a need in the art for a needle holder including a rotatable finger grip flange to provide alignment of the needle bevel relative to the skin, which does not require the medical professional to apply forceable action with both hands in order to effect rotation of the finger grip about the body of the needle holder.

### SUMMARY OF THE INVENTION

The present invention provides a needle holder including: an elongate tubular body having an open end for receiving a specimen collection tube; an opposed closed end for supporting a needle and a cylindrical wall therebetween; and a finger grip flange rotatively supported about the cylindrical wall at the open end of the body, characterized in that the tubular body includes a radially outwardly extending flange portion at the open end and at least one outwardly extending shoulder spaced from the fixed flange portion for longitudinally captivating the finger grip flange therebetween.

### BRIEF DESCRIPTION OF THE DRAWINGS

Fig. 1 is a perspective view of the needle holder of the present invention.

Fig. 2 is another perspective view of the needle holder of Fig 1.

Fig. 3 is an enlarged sectional top view of the needle holder of Figs. 1-2.

### DETAILED DESCRIPTION OF THE INVENTION

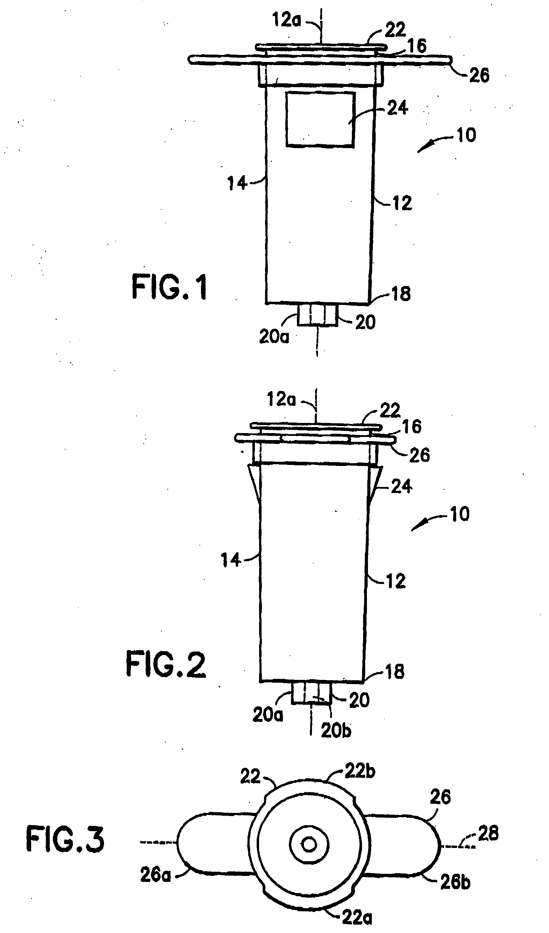

The present invention provides a needle holder including a rotatable finger grip flange. Needle holder **10** of the present invention is shown in Figs. 1 and 2 having an elongate tubular body **12.** Body **12** includes a generally cylindrical wall **14** which may be slightly tapered from an open end **16** to an opposed closed end **18.** Open end **16** is for receiving a specimen collection tube, such as a blood collection tube. The generally closed end **18** supports needle securement member 20 thereat for supporting and securing a needle (not shown) therein in conventional fashion.

Needle securement member **20,** which may be conical in shape, is preferably integrally mounted to closed end **18** for accommodating the connecting end socket of a hollow needle (not shown). Securement member **20** includes a neck portion **20a** having a central aperture **20b.** The aperture may be internally screw-threaded so as to accommodate an externally threaded base of a needle (not shown) which is inserted therein. The needle includes a beveled free end which is useful for piercing a patient's skin. Attachment of the needle to the securement member **20** fixes the rotated position of the beveled free end.

Body **12** includes a radially outwardly extending fixed flange portion **22** at open end **16** which may be integrally formed with body **12.** In one embodiment, fixed flange portion **22** includes two diametrically opposed tabs **22a** and **22b** which extend from body **12.** These tabs may be generally trapezoidal in shape or lozenge-shaped.

Further included along body **12** is a pair of outwardly extending diametrically opposed shoulders **24** spaced from flange portion **22**. In the embodiment shown in Figs. 1-2, shoulder **24** is in the shape of a tapered ramp, with the ramp tapering towards the closed end **18** of body **12.** Other configurations and number of shoulders are within the contemplation of the present invention.

As described above, a problem associated with prior art needle holders having a fixed finger grip flange has been the need for the medical professional to rotate a sheath-covered needle empirically relative to the fixed finger grip at the open end of the needle holder to obtain the correct orientation of the needle bevel plane with respect to the fixed flange, wherein the correct orientation would often only be obtained after multiple passes.

The present invention solves a need by providing a needle holder which allows the user to rotate the finger grip flange relative to the needle to obtain the correct orientation between the needle bevel and the finger grip flange in a single pass. For example, with reference to Figs. 1-3, body **12** of needle holder **10** includes a finger grip flange which is rotatable about cylindrical wall **14** at the open end **16** of body **12.** The rotatable finger grip flange **26** is held by friction fit between fixed flange portion **22** at open end **16** and shoulder **24.**

As may be appreciated, the rotatable finger grip of the needle holder of the present invention may be separately formed from plastic as a component of the needle holder and engaged in position around the tubular body by first being placed around the closed end **18** of body **12** and thereafter slid over the length of body **12** up and over shoulder 24, where it is then retained by friction fit between one or more of shoulders **24** and fixed flange **22** at open end **16.** As described above, shoulders **24** are desirably ramped to facilitate accommodation and longitudinal capivation of finger grip flange **26** between shoulders **24** and fixed flange **22.** The shoulders **24** and flange **22** define a space **27** wherein the flange is accommodated. The relative dimension between the space **27** and flange **26** is such that the flange is frictionally rotatable therein. This allows the flange to be easily, but not freely, rotated. Thus, the flange will remain, under friction, in the rotational position selected.

With reference now to Fig. 3, in one embodiment of the invention, the rotatable finger flange **26** includes two diametrically opposed tabs or wings **26a** and **26b** extending from cylindrical wall **14.** These opposed tabs of the rotatable finger grip flange **26** may be generally trapezoidal in shape, U-shaped or lozenge-shaped. In preferred embodiments, finger grip flange **26** extends farther from cylindrical wall **14** than does fixed flange portion **22.**

The bevel of a needle should face away from the skin to provide a large improvement in the skin-end blood vessel membrane-piercing capability of the needle. The needle holder of the present invention solves a need by providing the user with a rotatable finger grip flange to facilitate the user in obtaining the correct orientation of the needle bevel relative to the patient's skin, with the bevel facing away from the skin. With reference to Figs. 1-3, to effect rotation, a phlebotomist would hold body **12** with one hand and use the thumb and forefinger from the other hand to rotate finger grip flange **26** in order to properly orient the needle bevel. For example, the user may rotate finger grip flange **26** so as to bring the longitudinal axis **28** of finger grip flange **26** parallel to the needle tip's bevel plane, which typically makes an angle of usually 20-30 degrees with respect to longitudinal axis **12a** of tubular body **12.** The user only needs to apply forcible action with one hand to effect rotation. Therefore, this presents a significant advantage over prior art devices having rotatable finger grips which required the user to apply forcible action with both hands to effect rotation of the finger grip. With the present invention, once the needle has been placed onto securement member **20** and the sheath is removed, it is a straightforward process to bring the needle bevel into the correct orientation with respect to the patient's skin. Moreover, in contrast to prior art needle holders having a fixed finger grip flange at the open end of the holder, with the present needle holder, the user does not need to manipulate the needle before piercing a patient's skin by rotation of a connecting end socket of a needle for proper alignment of the needle bevel. For example, with the device of the present invention, once the needle bevel has been properly aligned by rotation of the finger grip flange, the fixed flange portion is then used as a finger abutting and guiding surface when positioning the bevel plane of the needle away from the plane of the patient's skin and to allow minimum inclination of the needle holder relative to the patient's skin to obtain optimum skin piercing.

During the skin piercing step, the rotatable finger grip will not rotate due to the friction fit of the rotatable finger grip between the fixed flange at the open end of the needle holder and the shoulder spaced from the fixed flange. This friction fit allows for the proper orientation of the needle with respect to the patient's skin to be maintained throughout the entire piercing step.

The preferred material for the rotatable finger grip and the cylindrical body of the needle holder including the fixed flange is a translucent plastic material. However, it is anticipated that the needle holder may be made of other materials, including glass, metal, and plastic and metal combinations.

The needle holder of the present invention eliminates the need for the medical professional to have to touch the needle socket to properly orient the needle with respect to the patient's skin and, thus, presents a significant advantage over prior art needle holders having a fixed finger grip flange.

Various other changes and modifications may be effected therein by one skilled in the art without departing from the scope and spirit of the invention, and is intended to claim all such changes and modifications as fall in the scope of the invention.

## Claims

1. A needle holder (10) comprising:
an elongate tubular body (12) having an open end (16) for receiving a specimen collection tube, an opposed closed end (18) for supporting a needle, and a cylindrical wall (14) therebetween; and
a finger grip flange (26) rotatively supported about said cylindrical wall at said open end of said body,
**characterized in that**,
said tubular body including a radially outwardly extending flange portion (22) at said open end and at least one outwardly extending shoulder (24) spaced from said flange portion for longitudinally captivating said finger grip flange therebetween.

2. The needle holder (10) of claim 1, wherein said finger grip flange (26) comprises two diametrically opposed tabs (26a, 26b).

3. The needle holder (10) of claim 1 or 2, wherein said finger grip flange (26) is formed of plastic.

4. The needle holder (10) of one of the claims 1-3, wherein said rotatively supported finger grip flange (26) extends radially outwardly from said tubular body (12) to a greater extent than said fixed flange portion (22).

5. The needle holder (10) of one of the claims 1-4, wherein said flange portion (22) of said tubular body (12) comprises two diametrically opposed tabs (22a, 22b).

6. The needle holder (10) of one of the claims 1-5, wherein said flange portion (22) is integrally formed with said tubular body (12) at said open end (16).

7. The needle holder (10) of one of the claims 1-6, wherein said tubular body (12) is formed of plastic.

8. The needle holder (10) of one of the claims 1-7, wherein said tubular body (12) conically tapers from said open end (16) to said closed end (18).

9. The needle holder (10) of one of the claims 1-8, wherein said shoulder (24) is ramped for snap-fit engagement of said finger grip flange (26) about said cylindrical wall (14) of said tubular body (12).

10. The needle holder (10) of one of the claims 1-9, wherein said friction fit prevents rotation of said finger grip flange (26) about said cylindrical wall (14) of said tubular body (12) when a user is piercing a patient's skin.

11. The needle holder (10) of one of the claims 1-10, wherein said body (12) comprises a needle securement member (20) at said closed end (18) for attaching said needle to said holder.

12. The needle holder (10) of claim 11, wherein said needle securement member (20) is conical in shape.

13. The needle holder (10) of claim 11 or 12, wherein said needle securement member (20) comprises an internally threaded aperture (20b).
